# EUROPEAN PATENT SPECIFICATION

(11) **EP 0 343 402 B1**
(45) Date of publication and mention of the grant of the patent: **30.11.1994**
(21) Application number: 89107917.0
(22) Date of filing: 02.05.1989
(51) Int. Cl.: A61N 1/05

(54) **Connector pin assembly and method of fabrication.**
Steckverbindung mit einem Steckerstift und zugehöriges Herstellungsverfahren.
Assemblage avec connecteur à ergot et méthode de fabrication associée.

(30) Priority: 25.05.1988 US 198539; 25.05.1988 US 198540
(43) Date of publication of application: 29.11.1989
(73) Proprietor: MEDTRONIC, INC., Minneapolis, Minnesota 55432-3576 (US)
(72) Inventor: Doan, Phong D., Shoreview Minnesota 55126 (US); Cobian, Kenneth E., St. Anthony Minnesota 55418 (US); Upton, James E., New Brighton Minnesota 55112 (US); Haeg, Daniel C., Brooklyn Park Minnesota 55444 (US); Hess, Douglas N., Maple Grove Minnesota 55369 (US); Bass, Wayne R., Coon Rapids Minnesota 55433 (US); Ufford, Keith A., Maple Grove Minnesota 55369 (US)
(74) Representative: Kopacz, William James

## Description

### BACKGROUND OF INVENTION

The present invention relates to medical electrical leads generally, and more particularly to cardiac pacing leads.

Early pacing leads, such as those disclosed in U.S. Patent No. 3,348,548 and U.S. Patent No. 3,788,329 employed separate conductor coils in a side-by-side or coaxial configuration, insulated from one another by plastic sheaths which covered the coils. More recently, multifilar coiled conductors having individually insulated coil wires have been pursued, as disclosed in Canadian Patent No. 1,146,228 for a MULTIPOLAR PACING CONDUCTOR, issued May 10, 1983 to Upton. This patent discloses a single, multiconductor DBS coil having individually insulated wires.

### SUMMARY OF THE INVENTION

The present invention is directed toward optimizing the construction of a pacing lead or other medical electrical lead of the type employing multiple, mutually insulated conductors, arranged in the form of a multipolar coil. In particular, the invention is directed toward improved connector assemblies and ring electrode assemblies for such leads.

Typically, in pacing leads employing a plurality of conductors, each of which are formed as a separate coil, connection of the coil to an electrode or a connector pin has been accomplished by means of crimping or swaging these components to one another, as disclosed in U.S. Patent No. 4,258,725, issued to O'Neill and U.S. Patent No. 4,328,812, issued to Ufford et al. However, where the conductors take the form of a multifilar coil, additional problems are presented. The conductors must be split off from the coil individually, and routed to the desired connector surface or electrode surface, without compromising the mechanical integrity of the lead at that point. Because one of the advantages of a multiconductor coil is that it allows fabrication of a smaller diameter lead, it is especially desirable to avoid unnecessary bulk in the vicinity of these assemblies.

Other prior art includes US 4,469,104 and 4,572,605

The present invention addresses these problems by providing connector and electrode assemblies which are constructed to allow all electrical connections to the conductors to be made by welding. This allows for the elimination of crimping or swaging cores commonly used in prior art leads. In addition, the connector and ring electrode assemblies are designed to provide increased structural integrity and improved sealing due to the use of hot-melt and adhesive backfill procedures during manufacture.

The present invention includes a connector pin assembly, having an elongated conductive connector pin having a proximal end and a distal end, a first insulative sheath coaxially mounted around said connector pin, the first insulative sheath terminating proximal to the distal end of the connector pin, a first connector ---ring mounted around the first insulative sheath, the first connector ring having a proximal end, a distal end, the distal end of said first connector ring terminating proximal to the distal end of the connector pin, a first conductive extension electrically and mechanically coupled to the distal end of the first connector ring and extending distally therefrom, the conductive extension extending distal to the distal end of the connector pin, a first electrical conductor coupled to the distal end of the connector pin, and a second conductor electrically and mechanically coupled to the distal end of said first conductive extension.

The invention also includes a method of fabricating the connector pin assembly.

### BRIEF DESCRIPTION OF THE DRAWINGS

Fig. 1 shows a plan view of a bipolar cardiac pacing lead according to the present invention.

Fig. 2 shows a sectional view of the lead of Fig. 1 in the vicinity of the connector assembly.

Fig. 3 shows an alternative connector assembly for use with a tripolar lead.

### DETAILED DESCRIPTION OF THE DRAWINGS

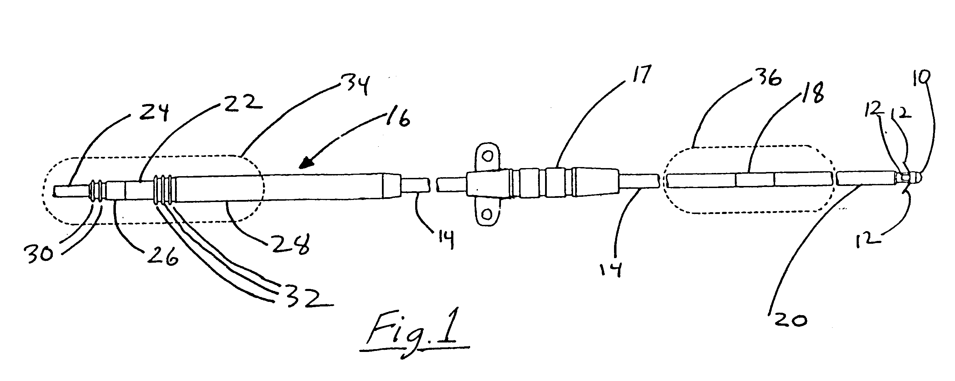

Fig. 1 is a plan view of a pacing lead employing improvements according to the present invention. At the distal end of the lead is located a pacing electrode 10, which may be fabricated according to any of a variety of well known electrode types. Preferably, the electrode is fabricated according to the disclosure in U.S. Patent No. 4,506,680, issued to Stokes, and incorporated herein by reference in its entirety. Electrode 10 is held in place within the heart by means of pliant tines 12, which are more fully described in U.S. Patent No. 3,902,501, issued to Citron et al, also incorporated herein by reference in its entirety.

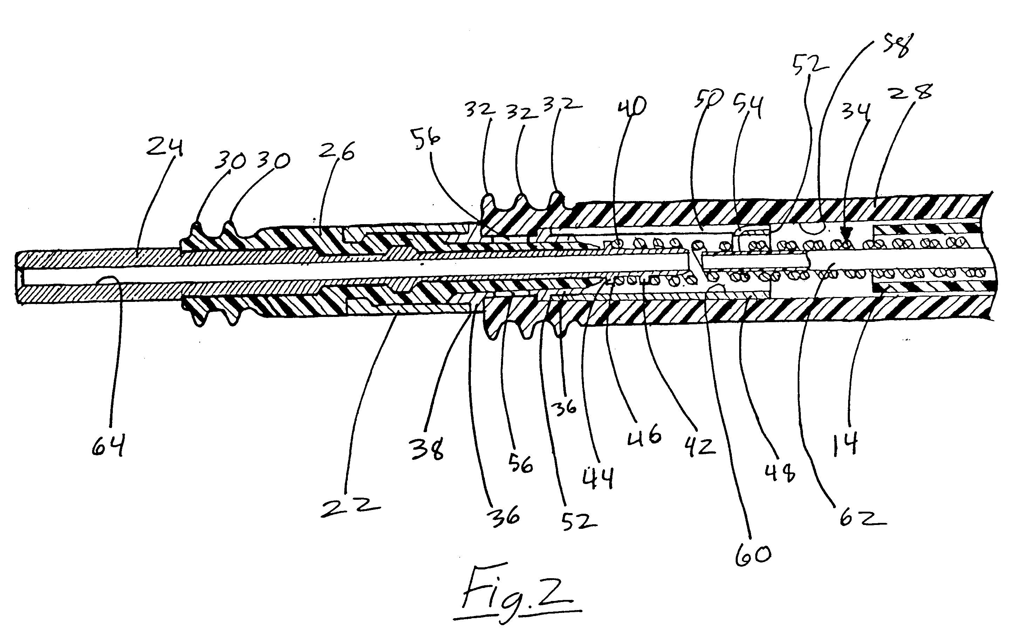

The lead includes an elongated insulative sheath 14, which extends from the connector assembly 16 to the ring electrode 18. The lead also includes a second insulative sheath 20, extending from ring electrode 18 to electrode 10. Mounted around insulative sheath 14 is a suture sleeve 17, which is used to anchor the lead at the point of venous insertion. Connector assembly 16 includes a connector pin 24 and a connector ring assembly comprising an exposed ring shaped member 22. Insulative sleeve 26 separates pin 24 from ring member 22. Insulative sleeve 28 extends distal to ring member 22. The connector assembly is provided with resiliant sealing rings 30 and 32, which seal the connector assembly within a corresponding receptacle mounted to a cardiac pacemaker. The area of the lead enclosed by dashed line 34 is illustrated in more detail in Fig. 2.

Insulative sleeves 26 and 28 are preferably fabricated of silicone rubber. Insulative sheaths 14 and 20, are preferably fabricated of polyurethane or silicone rubber. Connector pin 24, ring member 22, ring electrode 18 and tip electrode 10 are preferably fabricated of inert conductive metals such as platinum, Elgiloy® alloy, MP35N or stainless steel.

Fig. 2 shows a sectional view of the proximal portion of connector assembly 16, illustrated in Fig. 1. This view shows the interconnection of the multiconductor coil 34 with the other components of connector assembly 16, illustrated in Fig. 1. Components visible in Fig. 1 are labeled similarly in Fig. 2. Multiconductor coil 34 includes a first coiled conductor coupled to connector pin 24 and a second coiled conductor electrically coupled to ring member 22.

The connector assembly is fabricated by first laser welding ring member 22 to cylindrical member 36 by means of a circumferential laser weld at 38 to form a connector ring assembly. Assembled ring member 22 and cylindrical member 36 are assembled over connector pin 24, placed into a mold, and insulative sleeve 26 is then injection molded between them. This process is disclosed in more detail in U.S. Patent No. 4,572,605, issued to Hess.

The completed assembly of connector pin 24, insulative sleeve 26, ring member 22 and tubular member 36 is then coupled to one conductor 40 of multiconductor coil 34. Conductor 40 is screwed onto the distal end of connector pin 24, with protrusion 42 acting as a screw thread. Conductor 40 is screwed onto connector pin 24 until its proximal end butts up against circular flange 44. Conductor 40 is then coupled to circular flange 44 at 46 by means of a spot laser weld. The spacing intermediate circular flange 44 and protrusion 42 allows for a limited amount of strain relief immediately distal to the spot laser weld at 46.

Tubular extension 48, which takes the form of a cylinder having an extended longitudinal slot 50 is then slid over the distal end of cylindrical member 36 and coupled to it by means of a circumferential laser weld seen at section 52. Shallow grooved section 52, having a groove that corresponds generally to the size of conductor 54, is located at the proximal end of slot 50 in tubular extension 48. Conductor 54 is stripped of insulation and laid lengthwise in the grooved area 52, and laser welded to extension 48. Following this step, insulative sleeve 28 is slid over extension 48 and over cylindrical member 36. Member 36 is provided with a cross bore 56, which may be filled with medical adhesive, thereby bonding insulative sleeve 28 to insulative sleeve 26.

Finally, the entire assembly is backfilled with adhesive injected between insulative sheath 14 and insulative sleeve 28, filling the area between insulative sleeve 28 and sheath 14, as well as the lumen 58 of sleeve 28 and the lumen 60 of tubular extension 48. This serves to bond the components of the connector assembly to one another and to insulative sleeve 28 and to electrically insulate the conductor 40 and connector pin 24 from the conductor 54. For the sake of clarity, the backfilled adhesive is not shown in this illustration. Mounted within multiconductor coil 34 is a Teflon® plastic liner 62, which serves as a passageway for a stylet. The internal lumen of liner 62 is aligned with the internal bore 64 of connector pin 24.

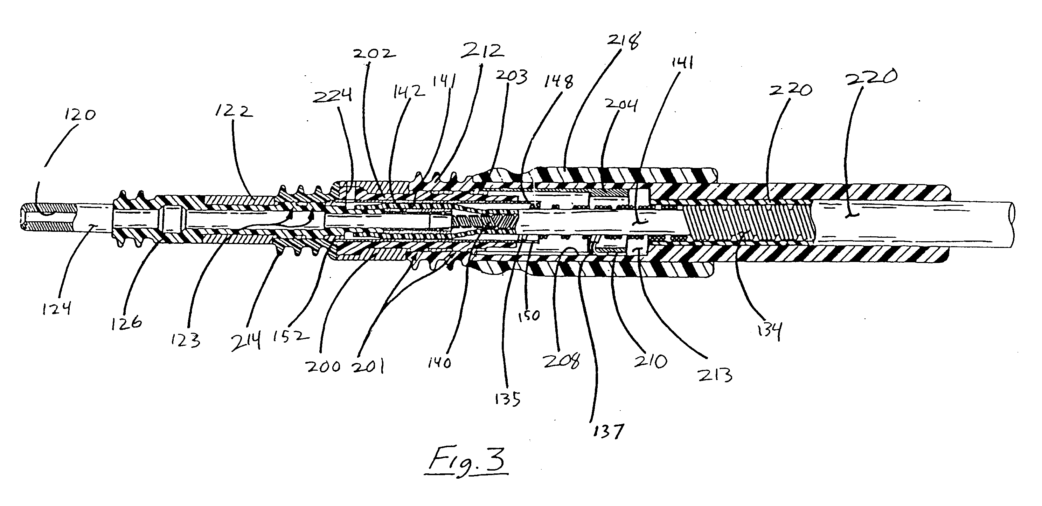

Fig. 3 shows an alternative connector assembly adapted for use with a tripolar, coaxial lead. This figure illustrates how sequential application of the basic techniques disclosed in conjunction with the bipolar lead can be used to produce a tripolar or multipolar connector having sequential, ring shaped connector surfaces.

The connector assembly of Fig. 3 is fabricated by first assembling connector ring 122 over connector pin 124, placing them in a mold and subsequently injection molding insulative sleeve 126 between them. This process is disclosed in the above cited Hess patent. Connector pin 124 is provided with a lumen 120 through which a stylet may be inserted. Connector ring 122 is provided with two perpendicular bores 123 which are filled by sleeve 126 during the molding procedure. The assembly of connector pin 124, sleeve 126 and connector ring 122 is then coupled to the inner conductor coil 140. Conductor coil 140 is slipped over the distal end of connector pin 124, and held in place by means of crimping sleeve 142. Insulative sleeve 141 is then slid over inner conductor 140.

After attachment of inner conductor 140 to the distal end of connector pin 124, tubular extension 148, which takes the form of a cylinder having a longitudinal slot 150 at its distal end and second connector ring 200 are assembled in their respective positions in an injection mold, and insulative sleeve 202 is injection molded between them. Connector ring 200 is provided with two perpendicular bores 201 which are filled by sleeve 202 during the molding process. This assembly is then slid over the distal end of connector ring 122 and coupled to it by means of a circumferential laser weld at 152. After this step, the proximal ends of two of the individual conductor wires 135 of multifilar coil 134 are stripped of insulation and laser welded to tubular extension 148 in slot 150. Following this step, a second tubular extension 204 is slid over tubular extension 148. Second tubular extension 204 is welded to second connector ring 200 by means of a circumferential laser weld at 203. Tubular extension 204 includes an elongated slot 208 and a shallow grooved section 210 at its distal end. The proximal ends of the two other conductors 137 from multipolar coil 134 are stripped, laid in shallow groove 210 and laser welded within groove 210. Outer insulative sheath 220 is then slid over multipolar coil 134.

After these steps have been completed, a sealing ring/strain relief member 212 is applied around the second connector ring 200. Because of the penetration of the material of sleeve 202 through bores 201, member 212 can be bonded directly to insulating sleeve 202 using a suitable adhesive. This provides a better bond than would be available if member 212 were bonded directly to connector ring 200. If sleeve 202 and sealing ring member 212 are both fabricated of silicone rubber, a silicone medical adhesive would be appropriate.

The interior of the assembly is backfilled with silicone adhesive 222 through bore 224, which extends through second connector ring 200, sleeve 202 and tubular extension 148. Backfilling continues until the adhesive is visible within the lumen of sealing ring/strain relief member 212, distal to tubular extension 204. This seals the lead, provides additional electrical insulation, and internally bonds the components together for added mechanical strength.

A second sealing ring member 214 is applied exterior to first connector ring 122. Because sleeve 126 extends through bores 123, sealing ring member 214 may be bonded directly to sleeve 126, using a suitable adhesive.

Surrounding sealing ring strain relief member 214 is a locking sleeve 218, which is intended to be used in conjunction with a deflectable beam locking member on the connector housing into which the lead is inserted.

Connector rings 122 and 200, connector pin 124, tubular extensions 148 and 204 and crimp sleeve 142 may all be fabricated of an inert, biocompatible metal such as stainless steel. Sleeves 126 and 202 and sealing ring members 212 and 214 are preferably fabricated of silicone rubber. Sheaths 141 and 220 and locking sleeve 218 are preferably fabricated of polyether urethane.

By repeated duplications of the connector rings, insulating sleeves and tubular extensions, a connector assembly having any number of linearily arranged connector rings may be produced. The particular configuration of the components allows for the use of laser or resistance welding to accomplish all metal to metal connections, leading to increased reliability and improved reproducibility during production. Therefore, both the method of fabrication of this connector assembly and the resultant product are believed to be a substantial improvement over previous in-line connector designs.

## Claims

1. A method of fabricating an in-line, multipolar electrical connector, including the steps of:
selecting an elongated conductive connector pin (24) having a proximal end and a distal end and a multipolar conductor wire (34) including at least first (40) and second (54) conductors;
mounting a conductive first connector ring (22,36) and a first insulative sleeve (26) around said connector pin, such that said first insulative sleeve separates said connector pin from said first connector ring (36), the method characterized in that the distal end of said first connector ring is proximal to the distal end of said connector pin and further characterized by the steps of
electrically coupling said first conductor to the distal end of said connector pin;
after said coupling step, mounting a conductive first extension (48) to the distal end of said connector ring, said conductive extension extending distal to the distal end of said connector pin; and
after said step of mounting said extension (48) to said connector ring (22,36), electrically coupling at least said second conductor (54) to the distal end of said conductive extension (48).

2. A method according to claim 1 wherein said step of electrically coupling said second conductor (54) to said first extension (48) comprises laser welding said second conductor to the distal end of said first extension.

3. A method according to claim 1 wherein said step of electrically coupling said first conductor (40) to said connector pin (24) comprises laser welding said first conductor to the distal end of said connector pin.

4. A method according to claim 1 or claim 2 or claim 3 wherein said step of attaching said first extension (48) to said connector ring (22,36) comprises laser welding said first extension to said first connector ring.

5. A method according to claim 3 wherein said step of selecting a connector pin comprises selecting a connector pin having a protrusion (42) extending perpendicular to said connector pin, near the distal end of said connector pin and wherein said at least one of said plurality of conductors takes the form of a conductor coil (40), and wherein said step of electrically coupling said first conductor to said connector pin (24) comprises screwing said at least one of said conductors over the distal end of said connector pin, using said protrusion as a screw thread, followed by spot welding said first conductor to said connector pin.

6. A method according to claim 1 further comprising the steps of:
mounting a second connector ring (200) and a second insulative sleeve (202) around said first extension (148), said second insulative sleeve separating said second connector ring from said first connector ring (122) and said first extension, said second connector ring having a proximal end and a distal end, the distal end of said second connector ring terminating proximal to the distal end of said first extension;
after said step of coupling said second one (135) of said plurality of conductors to said distal end of said first extension, coupling a second extension (204) to the distal end of said second connector ring, extending distally therefrom; and
coupling at least a third (137) one of said plurality of conductors to the distal end of said second extension sleeve.

7. A method according to claim 1 comprising the further step of back-filling between said first extension (48) and said connector pin (24) with an adhesive.

8. A connector pin assembly, comprising :
an elongated conductive connector pin (24) having a proximal end and a distal end;
a first insulative sheath (26) coaxially mounted around said connector pin, said first insulative sheath terminating proximal to the distal end of said connector pin (24);
a first connector ring (22,36) mounted around said first insulative sheath (26), said first connector ring having a proximal end a distal end, the distal end of said first connector ring terminating proximal to the distal end of said connector pin;
a first conductive extension (48) electrically and mechanically coupled to the distal end of said first connector ring and extending distally therefrom, said conductive extension extending distal to the distal end of the connector pin;
a first electrical conductor coupled (40) to the distal end of said connector pin; and
a second conductor (54) electrically and mechanically coupled to the distal end of said first conductive extension.

9. A connector assembly according to claim 8 further comprising an outer, insulative sheath (14) extending distally from said connector ring, enclosing the distal end of said first connector ring (22), said first extension (48) and said first (40) and second (54) conductors.

10. A connector assembly according to claim 8 or claim 9 further comprising an adhesive back-filled between and coupled to said connector pin (24) and said first extension (48).

11. A connector assembly according to claim 8, further comprising a second insulative sleeve (202) mounted to and surrounding the distal end of said first connector pin (124) and the proximal portion of said first conductive extension (48)(148);
a second connector ring (200) mounted to and surrounding the distal portion of said second insulative sleeve (202), said second connector ring (200) having a proximal end and a distal end;
a second conductive extension (204) coupled to the distal end of said second connector ring (200) and extending distally therefrom; and
a third electrical conductor (137) electrically and mechanically coupled to the distal end of said second extension.

12. A connector assembly according to claim 11, further comprising an insulative sheath (28,212) enclosing said second extension and said first (40), second (54) and third (137) conductors.

## Patentansprüche

1. Verfahren zum Herstellen eines mehrpoligen elektrischen In-line-Verbinders, mit den folgenden Schritten:
- Wählen eines langgestreckten, leitenden Verhinderstifts (24) mit einem proximalen Ende und einem distalen Ende sowie eines mehrpoligen Leiterdrahts (34) mit mindestens einem ersten (40) und einem zweiten (54) Leiter;
- Anordnen eines ersten leitenden Verbinderrings (22, 36) und einer ersten Isoliermanschette (26) um den Verbinderstift in solcher Weise, daß diese erste Isoliermanschette den Verbinderstift vom ersten Verbinderring (36) abtrennt; welches Verfahren **dadurch gekennzeichnet** ist, daß das distale Ende des ersten Verbinderrings proximal zum distalen Ende des Verbinderstifts angeordnet wird;
und ferner durch folgende Schritte gekennzeichnet ist:
- elektrisches Verbinden des ersten Leiters mit dem distalen Ende des Verbinderstifts;
- Anbringen, nach dem Verbindungsschritt, einer ersten leitenden Verlängerung (48) am distalen Ende des Verbinderrings, welche leitende Verlängerung sich distal zum distalen Ende des Verbinderstifts erstreckt; und
- elektrisches Verbinden, nach dem Schritt des Anbringens der Verlängerung (48) am Verbinderring (22, 36), zumindest des zweiten Leiters (54) mit dem distalen Ende der leitenden Verlängerung (48).

2. Verfahren nach Anspruch 1, bei dem der Schritt des elektrischen Verbindens des zweiten Leiters (54) mit der ersten Verlängerung (48) ein Laserverschweißen des zweiten Leiters mit dem distalen Ende der ersten Verlängerung beinhaltet.

3. Verfahren nach Anspruch 1, bei dem der Schritt des elektrischen Verbindens des ersten Leiters (40) mit dem Verbinderstift (24) ein Laserverschweißen des ersten Leiters mit dem distalen Ende dieses Verbinderstifts beinhaltet.

4. Verfahren nach Anspruch 1 oder Anspruch 2 oder Anspruch 3, bei dem der Schritt des Befestigens der ersten Verlängerung (48) am Verbinderring (22, 36) ein Laserverschweißen der ersten Verlängerung mit dem ersten Verbinderring beinhaltet.

5. Verfahren nach Anspruch 3, bei dem der Schritt des Auswählens eines Verbinderstifts das Auswählen eines Verbinderstifts mit einer Verlängerung (42) beinhaltet, die sich rechtwinklig zum Verbinderstift nahe dem distalen Ende des Verbinderstifts erstreckt, und wobei der mindestens eine der mehreren Leiter die Form einer Leiterwendel (40) einnimmt, und bei dem der Schritt des elektrischen Verbindens des ersten Leiters mit dem Verbinderstift (24) das Aufschrauben des mindestens einen Leiters auf das distale Ende des Verbinderstifts beinhaltet, wobei der Vorsprung als Schraubgewinde verwendet wird, gefolgt von einem Punktverschweißen des ersten Leiters mit dem Verbinderstift.

6. Verfahren nach Anspruch 1, ferner mit den folgenden Schritten:
- Anordnen eines zweiten Verbinderrings (200) und einer zweiten Isoliermanschette (202) um die erste Verlängerung (148), wobei die zweite Isoliermanschette den zweiten Verbinderring vom ersten Verbinderring (122) und der ersten Verlängerung trennt, wobei der zweite Verbinderring ein proximales Ende und distales Ende aufweist, wobei das distale Ende des zweiten Verbinderrings proximal zum distalen Ende der ersten Verlängerung endet;
- Verbinden, nach dem Schritt des Verbindens des zweiten (135) der mehreren Leiter mit dem distalen Ende der ersten Verlängerung, einer zweiten Verlängerung (204) mit dem distalen Ende des zweiten Verbinderrings, die sich distal von diesem ausgehend erstreckt; und
- Verbinden mindestens eines dritten (137) der mehreren Leiter mit dem distalen Ende der zweiten Verlängerungsmanschette.

7. Verfahren nach Anspruch 1, mit dem weiteren Schritt des Vornehmens eines Hinterfüllens zwischen der ersten Verlängerung (48) und dem Verbinderstift (24) durch einen Kleber.

8. Steckverbindung mit Steckerstift, mit:
- einem langgestreckten, leitenden Verbinderstift (24) mit einem proximalen Ende und einem distalen Ende;
- einer ersten Isolierhülle (26), die koaxial um den Verbinderstift herum angeordnet ist und die proximal zum distalen Ende des Verbinderstifts (24) endet;
- einem ersten Verbinderring (22, 36), der um die erste Isolierhülle (26) herum angeordnet ist und ein proximales Ende und ein distales Ende aufweist, wobei das distale Ende dieses ersten Verbinderrings proximal zum distalen Ende des Verbinderstifts endet;
- einer ersten leitenden Verlängerung (48), die elektrisch und mechanisch mit dem distalen Ende des ersten Verbinderrings verbunden ist und sich distal ausgehend von diesem erstreckt, wobei sich diese leitende Verlängerung distal zum distalen Ende des Verbinderstifts erstreckt;
- einem ersten elektrischen Leiter (40), der mit dem distalen Ende des Verbinderstifts verbunden ist; und
- einem zweiten Leiter (54), der elektrisch und mechanisch mit dem distalen Ende der ersten leitenden Verlängerung verbunden ist.

9. Verbinderanordnung nach Anspruch 8, ferner mit einer äußeren Isolierhülle (14), die sich distal ausgehend vom Verbinderring erstreckt und das distale Ende des ersten Verbinderrings (22), die erste Verlängerung (48) und den ersten (40) und zweiten (54) Leiter umschließt.

10. Verbinderanordnung nach Anspruch 8 oder Anspruch 9, ferner mit einem Kleber, der durch Hinterfüllung zwischen den Verbinderstift (24) und die erste Verlängerung (48) gebracht und mit diesen verbunden ist.

11. Verbinderanordnung nach Anspruch 8, ferner mit:
- einer zweiten Isoliermanschette (202), die am distalen Ende des ersten Verbinderstifts (124) und am proximalen Teil der ersten leitenden Verlängerung (48) (148) angeordnet ist und diese umgibt;
- einem zweiten Verbinderring (200), der am distalen Teil der zweiten Isoliermanschette (202) angebracht ist und diese umgibt, und der ein proximales Ende und ein distales Ende aufweist;
- einer zweiten leitenden Verlängerung (204), die mit dem distalen Ende des zweiten Verbinderrings (200) verbunden ist und sich distal ausgehend von diesem erstreckt; und
- einem dritten elektrischen Leiter (137), der elektrisch und mechanisch mit dem distalen Ende der zweiten Verlängerung verbunden ist.

12. Verbinderanordnung nach Anspruch 11, ferner mit einer Isolierhülle (28, 212), die die zweite Verlängerung und den ersten (40), zweiten (54) und dritten (137) Leiter umschließt.

## Revendications

1. Procédé de fabrication d'un connecteur linéaire en ligne multipolaire comprenant les étapes suivantes :
- la sélection d'une broche allongée conductrice de connecteur (24) possédant une extrémité proximale et une extrémité distale et d'un fil de conducteur multipolaire (34) comprenant au moins une premier (40) et un second (54) conducteurs.
- le montage d'un premier anneau de connecteur conducteur (22, 36) et d'un premier manchon d'isolation (26) autour de ladite broche de connecteur de telle façon que ledit premier manchon isolant sépare ladite broche de connecteur dudit premier anneau de connecteur (36);
procédé caractérisé en ce que l'extrémité distale dudit premier anneau de connecteur soit proche de l'extrémité distale de ladite broche de connecteur et caractérisé, de plus, par les étapes suivantes :
- le couplage électrique dudit premier conducteur à l'extrémité distale de ladite broche de connecteur;
- après ladite étape de couplage, le montage d'une première extension conductrice (48) à l'extrémité distale dudit anneau de connecteur, ladite extension conductrice s'étendant à l'écart de l'extrémité distale de ladite broche de connecteur; et
- après ladite étape de montage de ladite extension (48) sur ledit anneau de connecteur (22, 36), le couplage électrique d'au moins ledit second conducteur (54) sur l'extrémité distale de ladite extension conductrice (48).

2. Procédé selon la revendication 1, selon lequel ladite étape de couplage électrique dudit second conducteur (54) à ladite première extension (48) comprend une soudure par laser dudit second conducteur sur l'extrémité distale de ladite première extension.

3. Procédé selon la revendication 1, selon lequel ladite étape de couplage électrique dudit premier conducteur (40) à ladite broche de connecteur (24) comprend un soudage par laser dudit premier conducteur sur l'extrémité distale de ladite broche de connecteur.

4. Procédé selon la revendication 1 ou 2 ou 3, selon lequel ladite étape de fixation de ladite première extension (48) audit anneau de connecteur (22, 36) comprend un soudage par laser de ladite première extension sur ledit premier anneau de connecteur.

5. Procédé selon la revendication 3, selon lequel ladite étape de sélection d'une broche de connecteur comprend la sélection d'une broche de connecteur possédant une protubérance (42) s'étendant normale à ladite broche de connecteur, près de l'extrémité distale de ladite broche de connecteur, selon lequel au moins un parmi ladite pluralité de conducteurs prend la forme d'un bobinage de conducteur (40) et selon lequel ladite étape de couplage électrique dudit premier conducteur à ladite broche de connecteur (24) comprend le vissage d'au moins un desdits conducteurs sur l'extrémité distale de ladite broche de connecteur à l'aide de ladite protubérance servant de filet de vis, suivie d'un soudage par point dudit premier conducteur sur ladite broche de connecteur.

6. Procédé selon la revendication 1, comprenant, de plus, les étapes suivantes :
- le montage d'un second anneau de connecteur (200) et d'un second manchon d'isolation (202) autour de ladite première extension (148), ledit second manchon d'isolation séparant ledit second anneau de connecteur dudit premier anneau de connecteur (122) et de ladite première extension, ledit second anneau de connecteur possédant une extrémité proximale et une extrémité distale, l'extrémité distale dudit second anneau de connecteur se terminant proche de l'extrémité distale de ladite première extension;
- après ladite étape de couplage dudit second (135) parmi ladite pluralité de conducteurs à ladite extrémité distale de ladite première extension, le couplage d'une seconde extension (204) à l'extrémité distale dudit second anneau de connecteur, s'étendant de façon distale; et
- le couplage d'au moins un troisième (137) parmi ladite pluralité de conducteurs à l'extrémité distale dudit second manchon d'extension.

7. Procédé selon la revendication 1, comprenant l'étape supplémentaire de remplissage par un adhésif de la zone entre ladite première extension (48) et ladite broche de connecteur (24).

8. Ensemble de broche de connecteur comprenant:
- une broche de connecteur allongée conductrice (24) possédant une extrémité proximale et une extrémité distale;
- une première gaine isolante (26) montée, de façon coaxiale, autour de ladite broche de connecteur, ladite première gaine isolante se terminant près de l'extrémité distale de ladite broche de connecteur (24);
- un premier anneau de connecteur (22, 36) monté autour de ladite première gaine isolante (26); ledit premier anneau de connecteur possédant une extrémité proximale et une extrémité distale, l'extrémité distale dudit premier anneau de connecteur se terminant proche de l'extrémité distale de ladite broche de connecteur;
- une première extension conductrice (48) couplée de façon électrique et mécanique à l'extrémité distale dudit premier anneau de connecteur et s'étendant de façon distale, ladite extension conductrice s'étendant proche de l'extrémité distale de la broche de connecteur;
- un premier conducteur électrique (40) couplé à l'extrémité distale de ladite broche de connecteur; et
- un second conducteur (54) couplé de façon électrique et mécanique à l'extrémité distale de ladite première extension conductrice.

9. Ensemble de connecteur selon la revendication 8, comprenant, de plus, une gaine externe d'isolation (14) s'étendant, de façon distale, dudit second anneau de connecteur, enfermant l'extrémité distale dudit premier anneau de connecteur (22), de ladite première extension (48) et desdits premier (40) et second (54) conducteurs.

10. Ensemble de connecteur selon la revendication 8 ou 9, comprenant, de plus, un adhésif de remplissage et couplé à ladite broche de connecteur (24) et à ladite première extension (48).

11. Ensemble de connecteur selon la revendication 8, comprenant, de plus :
- un second manchon d'isolation (202) monté sur et entourant l'extrémité distale de ladite première broche de connecteur (124) et la partie proximale de ladite première extension conductrice (148);
- un second anneau de conducteur (200) monté sur et entourant la partie distale dudit second manchon d'isolation (202), ledit second anneau de connecteur (200) possédant une extrémité proximale et une extrémité distale;
- une seconde extension conductrice (204) couplée à l'extrémité distale dudit second anneau de connecteur (200) et s'étendant de façon distale; et
- un troisième conducteur électrique (137) couplé, de façon électrique et mécanique, à l'extrémité distale de ladite seconde extension.

12. Ensemble de connecteur selon la revendication 11, comprenant, de plus, une gaine isolante (28, 212) enfermant ladite seconde extension et lesdits premier (40), second (54) et troisième (137) conducteurs.
